# FASCICULE DE BREVET EUROPEEN

(11) **EP 3 717 961 B1**
(45) Date de publication et mention de la délivrance du brevet: **30.09.2026**
(21) Numéro de dépôt: 18762367.3
(22) Date de dépôt: 03.08.2018
(51) Int. Cl.: G02B 30/24, G02B 30/25, A61B 5/16, G02B 27/02, G02C 7/10

(54) **DISPOSITIF OPTIQUE FACILITANT LA LECTURE**
OPTISCHE VORRICHTUNG ZUR ERLEICHTERUNG DES LESENS
OPTICAL DEVICE FOR FACILITATING READING

(30) Priorité: 30.11.2017 FR 1701260
(43) Date de publication de la demande: 07.10.2020
(73) Titulaire: Le Floch, Albert, 35700 Rennes (FR); Université de Rennes, 35042 Rennes (FR)
(72) Inventeur: LE FLOCH,, Albert, 35700 Rennes (FR); ROPARS, Guy, 35000 Rennes (FR)
(74) Mandataire: Novagraaf Technologies
(86) Numéro de dépôt international: PCT/FR2018/052011
(87) Numéro de publication internationale: WO 2019/106243

(56) Documents cités:
- FR-A1- 2 969 893
- FR-A3- 2 979 464
- US-A1- 2012 019 639
- US-A1- 2013 017 520
- US-A1- 2013 017 520
- LE FLOCH ALBERT ET AL: "Left-right asymmetry of the Maxwell spot centroids in adults without and with dyslexia", BIOLOGICAL SCIENCES, vol. 284, no. 1865, 18 October 2017 (2017-10-18), pages 20171380, XP093008640, ISSN: 0962-8452, Retrieved from the Internet <URL:https://royalsocietypublishing.org/doi/full-xml/10.1098/rspb.2017.1380> [retrieved on 20221213], DOI: 10.1098/rspb.2017.1380

## Description

### 1. Domaine de l'invention.

La présente invention concerne un dispositif optique. L'invention concerne plus particulièrement un dispositif optique de filtrage facilitant la lecture de contenus, notamment textuels, pour des personnes sujettes à la dyslexie.

### 2. Etat de l'art.

La dyslexie est communément définie comme un ensemble de troubles de la lecture qui apparaissent à l'enfance. Il s'agit de troubles spécifiques d'apprentissage dont les causes apparaissent complexes et ont fait et font toujours l'objet de nombreuses études dans des domaines variés.

Il est généralement exclu de considérer que les causes de la dyslexie puissent être uniquement d'ordre sensoriel, social ou psychologique.

Des études menées dans le domaine des neurosciences permettent de penser qu'il pourrait s'agir d'un trouble neurologique spécifique.

Les progrès réalisés dans le domaine de l'imagerie médicale ont pu mettre en évidence le rôle de certaines zones du cerveau dans les processus de lecture et de maîtrise du langage.

Les solutions apportées pour traiter les troubles de la dyslexie s'appuient sur des travaux et activités ludiques selon les difficultés propres à chaque sujet. L'objectif d'un tel accompagnement est d'apporter au sujet objet de troubles une autonomie en matière de lecture. Les méthodes connues sont développées autour de travaux dans des domaines tels que la psychologie, la psychomotricité et l'orthoptie, par exemple.

Récemment, des études ont été conduites, établissant une corrélation entre des particularités propres au mécanisme de la vision et la présence de troubles spécifiques de la dyslexie. La publication "Left-right asymmetry of the Maxwell spot centroids in adults without and with dyslexia (Le Floch A, Ropars G. 2017, Proc. R. Soc. B 284: 20171380, http://dx.doi.org/10.1098/rspb.2017.1380) mentionne le rôle des fovéas, situées dans l'œil humain, dans la construction des images perçues, au niveau cérébral, et le fait que des caractéristiques identiques ou sensiblement identiques pour chacun des yeux d'un même sujet entraînent chez lui des dysfonctionnements dans le processus de la vision et du traitement phonologique au niveau cérébral. La transmission d'une image miroir d'un hémisphère à l'autre, par exemple, perturbe sensiblement le processus de lecture d'éléments graphiques ou de contenus textuels chez des sujets présentant des troubles caractéristiques de la dyslexie. Des troubles liés à une instabilité de la fixation et/ou une instabilité posturologique, ou encore des défauts de convergence binoculaire liés aux muscles oculomoteurs, peuvent également entraîner des encombrements visuels comme l'effet miroir.
Le document FR2979464A3 décrit un dispositif d'affichage d'une pluralité de contenus et une pluralité de lunettes pour visualiser des contenus respectifs.

### 3. Résumé de l'invention.

La portée de la protection est définie par les revendications.

L'invention permet d'améliorer au moins certains des inconvénients de l'art antérieur en proposant un dispositif optique, de filtrage, adapté à faciliter la lecture de contenus, tels que des contenus graphiques, sur tous types de supports, et notamment sur un support papier ou sur un écran. Le dispositif proposé opère des périodes successives d'ouverture et de fermeture d'un espace ou plan de vision, au moins dans le spectre de la lumière visible. La lumière reçue via l'espace ou le plan de vision est ainsi transmise ou inhibée (voire supprimée) périodiquement, par un obstacle (filtre polarisant) mis en œuvre par le dispositif optique, selon des cycles successifs opérés à une fréquence prédéterminée. Les périodes successives d'ouverture de l'espace de vision ont chacune une durée comprise dans un intervalle de valeurs allant de 15 à 35% de la durée totale des cycles opérés.

Le dispositif optique selon l'invention comprend une unité de contrôle et un ou plusieurs (par exemple deux) modules de filtrage chacun adaptés à occulter (inhiber) périodiquement la lumière (en opérant une fermeture de l'espace de vision) dans un spectre de lumière visible. Le dispositif optique est configuré pour l'ouverture et la fermeture (ou l'inhibition) périodique d'un espace de vision traversé par de la lumière, dans un spectre de lumière visible, selon des cycles successifs opérés à une fréquence prédéterminée Fd. Chaque cycle présente une durée T et comprend une période d'ouverture de l'espace de vision de durée T1 suivie ou précédée d'une période de fermeture de l'espace de vision de durée T2. Le dispositif est configuré pour que la durée T1 des périodes d'ouverture de l'espace de vision soit comprise dans un intervalle de valeurs allant de 15 à 35% de la durée T des cycles.

Selon un mode de réalisation de l'invention, la fréquence Fd prédéterminée est définie par un utilisateur du dispositif optique de filtrage.

Un tel dispositif prend, par exemple, la forme d'une monture de lunettes comprenant, en lieu et place des habituels verres correcteurs, un ou plusieurs éléments filtrants comprenant des cristaux liquides contrôlables utiles au contrôle de la polarisation de la lumière qui leur est appliquée. Le dispositif comprend par exemple, une unité de contrôle miniaturisée, embarquée dans la monture, et adaptée au contrôle de l'orientation des cristaux liquides. Cette polarisation est variable dans le temps, sous contrôle de l'unité de contrôle précitée.

Selon une variante, le dispositif optique de filtrage prend la forme d'un plan filtrant adapté à être positionné sur ou devant un écran d'un dispositif de restitution graphique.

Avantageusement la fréquence Fd des cycles (par exemple de polarisation de la lumière reçue) ainsi opérés est comprise dans un intervalle de valeurs allant de 60 à 90 Hz, grâce à un système accordable fonctionnant alors en dispositif anti-encombrement visuel. Ce système met à profit les mécanismes hébbiens dans les neurones du cortex.

Selon un mode de réalisation de l'invention, la fréquence prédéterminée d'ouverture et de fermeture de l'espace de vision est sélectionnée de façon discrète, c'est-à-dire parmi une pluralité de fréquences prédéterminées dans l'intervalle de fréquences susmentionné.

Selon un mode de réalisation, la fréquence Fd varie dans le temps, afin de faciliter plus encore, dans certains cas, l'effacement de l'encombrement visuel et la stabilité binoculaire

Selon un mode de réalisation, la fréquence Fd varie en croissant par pas successifs jusqu'à une valeur maximale selon une première vitesse, dite vitesse de croissance, puis varie en décroissant par pas successifs jusqu'à une valeur minimale selon une seconde vitesse, dite vitesse de décroissance, les variations croissantes et décroissantes se répétant itérativement dans le temps.

Selon un mode de réalisation, ladite vitesse de décroissance et égale à ladite vitesse de croissance.

Selon un mode de réalisation, lesdits pas successifs sont de durées égales.

Selon un mode de réalisation, lesdits pas successifs varient en durée de sorte que la valeur de ladite fréquence Fd évolue selon une forme d'onde en triangle, en scie, ou en sinusoïde entre ladite valeur maximale et ladite valeur minimale.

Selon un mode de réalisation de l'invention, la durée T1 des périodes d'ouverture de l'espace de vision varie dans le temps.

Selon un mode de réalisation de l'invention, le module de filtrage comprend des éléments polarisants et des éléments de type cristaux liquides adaptés à une polarisation de la lumière reçue, transmise via un espace de vision.

Selon un mode de réalisation de l'invention, la fermeture réalisée de l'espace de vision dans un spectre de lumière visible est réalisée par l'orientation de cristaux liquides assemblés en une ou plusieurs surfaces (ou un ou plusieurs plans) dans le dispositif optique selon l'invention.

Les termes « espace de vision» sont à interpréter ici comme tout ou partie du champ de vision d'un sujet humain. Il peut s'agir du champ de vision d'un œil pris isolément ou de la combinaison du champ de vision des deux yeux, ou d'un sous-ensemble de l'un ou l'autre de ces variantes. Les termes « plan de vision » signifient alors une section de l'espace de vision tel que précédemment défini par un plan perpendiculaire à la direction moyenne de la lumière transmise via l'espace de vision, depuis un support observé et jusqu'à un œil ou jusqu'aux yeux d'un sujet.

Ainsi, l'«espace de vision » peut se définir comme une portion d'espace comprise entre les yeux d'un sujet humain et un objet visualisable par ce sujet, par l'un ou l'autre de ses yeux, ou les deux. Un « plan de vision » est à interpréter ici comme un plan définissant une coupe de cet espace.

Ainsi les termes « ouverture d'un espace de vision » ou « ouverture d'un plan de vision » sont à interpréter ici comme une transmission normale (non délibérément altérée ou inhibée par un artifice) de la lumière visible, transmise via l'espace de vision. A contrario, les termes « fermeture d'un espace de vision » ou «fermeture d'un plan de vision » sont à interpréter ici comme l'établissement et le maintien d'une barrière (d'un filtre ou d'un obstacle), plus ou moins opaque, à la transmission de la lumière visible, via l'espace de vision, pour une durée prédéterminée. Une alternance d'opérations d'ouvertures et de fermetures (d'inhibition ou d'atténuation de la lumière reçue) d'un même espace de vision constitue ainsi une opération de « filtrage » au sens de la présente description. Le terme « ouverture d'un espace de vision » peut donc être interprété comme une absence totale de filtrage et le terme « ouverture d'un espace de vision » peut donc être interprété comme une présence, au moins partielle, d'un filtrage.

Avantageusement, l'utilisation d'une gamme de fréquences des cycles d'ouverture et de fermeture de l'espace de vision, à partir de 60 Hz, permet de s'affranchir des effets connus de clignotements (ou scintillements) perceptibles par l'œil de l'être humain, la limite de perception du clignotement par l'œil se situant aux alentours de 60 Hz, pour l'être humain (hors considération des espèces animales).

Avantageusement, l'alternance de périodes d'ouverture et de fermeture de l'espace de vision dans le spectre de la lumière visible, orienté vers un support, permet une "focalisation" du cerveau d'un sujet regardant ce support, sur une image représentative du contenu représenté sur le support observé, puis, une disparition de cette même image de la vue du sujet avant qu'elle ne soit transmise sous forme d'image miroir entre un hémisphère cérébral et l'autre hémisphère cérébral de ce sujet regardant ce support. Le délai requis pour que le cerveau transmette une image, perçue par l'œil, entre un hémisphère et l'autre hémisphère, sous forme d'image miroir pour ce dernier, est de l'ordre de 10 ms.

Ainsi, le cerveau privilégie l'image transmise, à partir du support observé, par rapport à son image miroir, et la confusion existante chez le sujet qui présente une forte similarité des caractéristiques de ses deux fovéas, est moindre ou sensiblement diminuée pour la lecture du contenu (graphique et/ou textuel) représenté sur le support, à travers le dispositif optique de filtrage, notamment lorsque ce contenu est représentatif d'un ou plusieurs contenus textuels.

Avantageusement, l'utilisation d'un commutateur configuré pour obtenir sélectivement une ouverture permanente de l'espace de vision (vision « ordinaire » sans filtrage) et une ouverture de l'espace de vision telle que décrite ci-avant (alternance de périodes d'ouverture et de fermeture de l'espace de vision) permet à un utilisateur sujet à des troubles dyslexiques de comparer ses performances usuelles en lecture (correspondant à une vision ordinaire) à ses performances obtenues sous contrôle électronique du filtrage de l'espace de vision selon l'invention (alternance de périodes d'ouverture et de fermeture de l'espace de vision) après optimisation éventuelle de ses propres paramètres (paramètres de fréquence et de rapport cyclique permettant à l'utilisateur d'obtenir un meilleur confort de lecture).

Avantageusement, le dispositif optique de filtrage selon l'invention est fonctionnel avec de la lumière naturelle ou de la lumière polarisée. Selon un mode de réalisation de l'invention, le dispositif de filtrage opère simultanément un filtrage dans l'espace de vision ou le plan de vision de façon identique ou similaire pour les deux yeux d'un sujet l'utilisant.

L'invention concerne également une méthode de filtrage optique dans un dispositif optique, le dispositif comprenant une unité de contrôle et un module de filtrage optique adaptés à l'ouverture et à la fermeture d'un espace de vision dans un spectre de lumière visible, la méthode comprenant des ouvertures et fermetures périodiques d'un espace de vision par cycles successifs opérés à une fréquence prédéterminée, chaque cycle ayant une durée T et comprenant une période d'ouverture de l'espace de vision de durée T1 suivie ou précédée d'une période de fermeture de l'espace de vision de durée T2, dans laquelle la durée T1 des périodes d'ouverture de l'espace de vision est comprise dans un intervalle de valeurs allant de 15 à 35% de la durée T des cycles.

### 4. Liste des figures.

L'invention sera mieux comprise, et d'autres particularités et avantages apparaîtront à la lecture de la description qui va suivre, la description faisant référence aux dessins annexés parmi lesquels :
- la **figure 1** est un schéma de représentation d'un signal EL de commande (contrôle) d'un module de filtrage optique LIMOD selon un mode de réalisation particulier et non limitatif de l'invention.
- la **figure 2** est une représentation structurelle de l'architecture d'un dispositif optique FILT selon un mode de réalisation particulier et non-limitatif de l'invention.

### 5. Description détaillée de modes de réalisation de l'invention.

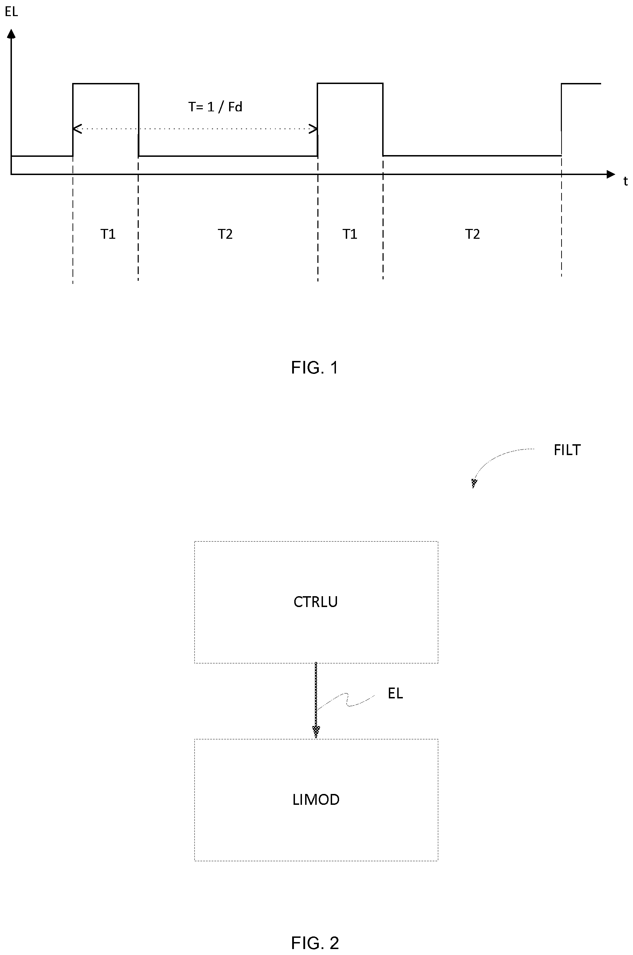

Sur la **figure 2****,** les modules représentés sont des unités fonctionnelles, qui correspondent ou non à des unités physiquement distinguables. Par exemple, ces modules ou certains d'entre eux sont regroupés dans un unique composant. *A contrario,* selon d'autres modes de réalisation, certains modules sont composés d'entités physiques séparées.

La **figure 1** est une représentation temporelle d'un signal EL de commande d'ouverture et de fermeture d'un espace ou plan de vision dans le spectre de la lumière visible, dans un dispositif optique de filtrage FILT, selon un mode de réalisation particulier et non limitatif de l'invention. Le signal EL varie selon le temps t et prend périodiquement deux états successifs. Selon le mode de réalisation préféré, une assertion du signal EL à l'état haut contrôle une ouverture de l'espace de vision à travers le dispositif optique FILT susceptible de transmettre la lumière visible, de manière globalement discontinue, d'un contenu graphique et/ou textuel à partir d'un support quelconque, tel qu'un livre ou un écran, par exemple.

Selon un raisonnement similaire, le signal EL contrôle la fermeture (obturation ou inhibition) de l'espace de vision lorsqu'il est positionné à l'état bas. Le signal EL est un signal périodique de fréquence prédéterminé Fd tel que Fd = 1 / (T1+T2). T1 est la période d'ouverture de l'espace ou plan de vision, dans le spectre de la lumière visible, soit une période de transmission de la lumière reçue depuis un support portant un motif représentant un contenu graphique et/ou textuel vers lequel l'espace de vision est orienté. T2 est la période, dite de fermeture de l'espace de vision, durant laquelle l'espace de vision est obturé (fermé) ou que la transmission de la lumière visible est interrompue ou inhibée, ou encore, en d'autres termes la période pendant laquelle le contenu graphique et/ou textuel présent sur un support vers lequel l'espace de vision est orienté n'est plus normalement perceptible par l'œil ou les yeux en effectuant une visualisation ou une lecture. Les termes "contenu graphique" sont à interpréter ici comme n'importe quel contenu représenté sur un support quelconque, notamment au format papier mais pas seulement, et constitué d'éléments élémentaires tels que, à titre d'exemple des points ou pixels juxtaposés, de sorte que le contenu représente des éléments de formes variées et notamment un ou plusieurs contenus textuels construits à partir de signes ou de symboles d'un ou plusieurs alphabets.

Ainsi un contenu textuel apposé sur un support correspond ici à un contenu interprétable dans un ou plusieurs langages, susceptible d'être lu et interprété par un sujet, utilisateur du dispositif optique de filtrage, positionné de sorte à ce qu'un sujet puisse regarder le support (livre ou écran, par exemple) pour une opération de lecture ou de visualisation. Un tel dispositif optique de filtrage est, à titre d'exemple, une paire de lunettes, un casque, un masque, un écran intercalaire, un plan filtrant configuré pour être disposé devant ou contre un écran de restitution. Cette liste d'exemples n'étant pas exhaustive.

Selon un mode de réalisation de l'invention, le rapport cyclique T1 / (T1+T2) entre les périodes d'ouverture et de fermeture (ou inhibition) de l'espace de vision, respectivement de durée T1 et T2, a une valeur comprise entre 15% et 35% du cycle, et la fréquence Fd de variation du signal EL est comprise entre 60 Hz et 90 Hz.

Préférentiellement, le rapport cyclique T1/(T1+T2) entre les périodes d'ouverture et de fermeture (ou inhibition) de l'espace de vision, respectivement de durée T1 et T2, a une valeur comprise entre 22% et 32% du cycle, et la fréquence Fd de variation du signal EL est comprise entre 70 Hz et 85 Hz.

Plus préférentiellement, la fréquence du signal est égale à 70 Hz ou 84 Hz et le rapport cyclique T1 / (T1+T2) est égal à 20%.

Avantageusement, le signal de contrôle EL peut être aisément forcé de façon prolongée dans son état associé à une activation du faisceau lumineux, ce qui correspond à un débrayage de la méthode de filtrage optique mise en œuvre dans le dispositif FILT selon l'invention. Il serait ainsi possible de ne pas mettre en œuvre la méthode de filtrage optique, dans un dispositif de filtrage selon l'invention, dans le cas où, pour un sujet non dyslexique, une gêne visuelle apparaitrait du fait de la discontinuité d'ouverture de l'espace de vision, et sans avoir alors à successivement retirer de / apposer dans l'espace de vision, le dispositif.

Avantageusement, il est possible d'affiner le réglage de la fréquence Fd dans l'intervalle de valeurs décrit dans le but d'adapter la période T à la sensibilité d'un utilisateur du dispositif optique FILT de filtrage, dans la gamme de fréquences indiquée. En effet, chaque individu possède une sensibilité propre en termes de vision et perçoit plus ou moins des variations de fréquence d'un faisceau lumineux. Ainsi, un réglage fin peut être rendu accessible à l'utilisateur par l'intermédiaire d'un bouton de réglage, d'un curseur, implémenté matériellement ou via une interface utilisateur quelconque (éléments graphiques d'un menu sur un écran de contrôle, par exemple, ou sur un dispositif de contrôle à distance).

**La** **figure** 2 est une représentation structurelle du dispositif optique FILT de filtrage selon un mode de réalisation particulier et non-limitatif de l'invention. Cette figure représente l'architecture globale du dispositif optique FILT, encore communément appelé « lunettes » ou « casque ». Le dispositif optique FILT comprend deux modules principaux qui sont une unité de contrôle CTRLU et un module de filtrage LIMOD. L'unité de contrôle CTRLU est le cœur du système en termes de contrôle et comprend un circuit bistable (ou hacheur) classique, adapté à la génération du signal EL. Le circuit de hachage bistable de l'unité de contrôle CTRLU délivre le signal EL caractérisé par la fréquence Fd et par son rapport cyclique T1/(T1+T2). Bien évidemment l'unité de contrôle CTRLU comprend tous les éléments usuels mis en œuvre dans une telle architecture, tels que, à titre d'exemples, un ou plusieurs amplificateurs opérationnels, des résistances et capacités, une ou plusieurs diodes, une alimentation (éventuellement sur batterie(s)), un circuit de remise à zéro, un circuit de supervision d'alimentation, une interface de puissance, un amplificateur de courant, la liste de ces éléments n'est pas exhaustive. Les détails architecturaux de l'unité de contrôle CTRLU ne sont pas décrits plus encore dans la mesure où ceux-ci ne sont pas utiles à la compréhension de l'invention. Selon un mode de réalisation de l'invention, le module CTRLU comprend un circuit bistable construit autour d'un amplificateur opérationnel, couplé à un circuit d'amplification de courant. Le module optique de filtrage LIMOD est un module comprenant une ou plusieurs surfaces comprenant des cristaux liquides polarisés dont la polarisation est commandée électriquement par signal électrique. Le module LIMOD est alors adapté au filtrage d'une lumière transmise dans le spectre de la lumière visible, ou sensiblement plus large. Avantageusement, l'espace de vision peut être plus ou moins filtré pour être alors configuré pour la lecture optimisée d'une surface plus ou moins grande. Une telle focalisation peut être réalisée par l'utilisation d'éléments optiques additionnels (lentilles) ou encore d'éléments mécaniques (diaphragmes, par exemple), ou les deux à la fois. Ainsi, le module de filtrage est par exemple implémenté sous la forme de montures de lunettes comprenant une ou des surfaces translucides, en lieu et place des verres correcteurs de lunettes correctrices, lesquelles surfaces sont porteuses d'éléments de type cristaux liquides pouvant être orientés par polarisation. Par exemple, une surface porteuse peut être configurée pour comprendre des vitrages entre lesquels sont pris en sandwich un cristal liquide nématique sous forme de gouttelettes immobilisées dans un polymère entre deux surfaces de verre recouverte d'une couche métallique fine pour constituer un condensateur. Le module de filtrage ainsi réalisé passe par exemple d'un état légèrement opaque sous tension à un état translucide en l'absence de tension.

Les cristaux liquides, de par leurs structures chimiques variables, peuvent se comporter comme des dipôles capables d'être orientés selon des lignes de champs, sous l'action d'un champ électrique.

De ce fait, le module de filtrage optique LIMOD peut opérer une variation de la directivité de lumière transmise dans l'espace de vision en créant un phénomène de directivité des rayons lumineux en provenance d'un support visualisé par un sujet. Les détails d'implémentation des éléments filtrants à cristaux liquides (éléments polarisants) ne sont pas décrits plus encore ici, car bien connus de l'homme du métier et n'étant pas utiles à la compréhension de l'invention.

Les termes « fermeture», « obturation », ou encore « inhibition» de la lumière transmise dans l'espace de vision décrit, lequel est orienté entre un support visualisé ou lu et les yeux d'un sujet, doivent être interprétés ici comme correspondant à une atténuation partielle ou totale de l'amplitude de lumière transmise, du fait du filtrage optique opéré par le mécanisme induit de directivité de la lumière perçue par le sujet, grâce à la polarisation effectuée par le module de filtrage optique LIMOD.

Avantageusement, le dispositif optique de filtrage FILT opère une polarisation verticale ou sensiblement verticale de la lumière reçue, transmise depuis un support visualisé ou lu. Ainsi, lorsque ce support est un écran ou un quelconque dispositif de restitution (de type afficheur) lui-même polarisé à +45°, 0°, ou -45° par rapport à la verticale, ce qui est fréquemment le cas pour les équipements de restitution de contenus visuels (TV, ordinateur, smartphone, par exemple), une transmission de la lumière entre le support visualisé et les yeux d'un sujet observant ce support reste toujours possible. Par opposition, et selon un raisonnement similaire, si le dispositif de filtrage optique FILT comprenait un module LIMOD polarisant la lumière reçue avec un axe de polarisation à +45° et utilisé pour la lecture d'un contenu sur un dispositif de restitution (écran, afficheur) polarisant la lumière émise avec un axe de polarisation de -45°, il y aurait obturation totale ou quasi-totale de la lumière transmise dans l'espace de vision, même en position d'ouverture du dispositif FILT.

C'est la capacité à procéder à des ouvertures et fermetures, successivement, de l'espace de vision entre un support de lecture visualisé et un sujet opérant la lecture, lequel support présente un ou plusieurs contenus graphiques et/ou textuels, sous contrôle du module bistable de l'unité CTRLU, qui permet avantageusement au cerveau d'un sujet de privilégier une image plutôt que son image miroir, perçue à partir du support lorsque ce dernier est observé via le dispositif optique de filtrage FILT selon l'invention. Avantageusement, cela permet d'aider conséquemment la lecture et le déchiffrage de contenus textuels, chez un sujet présentant des troubles dyslexiques.

Avantageusement, l'unité de contrôle CTRLU comprend en sortie un signal EL d'ouverture (ou de fermeture / obturation / inhibition) de l'espace de vision, connecté en entrée du module de filtrage optique LIMOD.

En d'autres termes, les variations du signal EL de contrôle du dispositif de filtrage optique LIMOD, opérées par l'unité de contrôle CTRLU comprenant un circuit bistable, à une fréquence prédéterminée Fd, permettent d'agir sur le filtrage de la lumière reçue depuis un support portant un contenu graphique. Ainsi, ce contenu graphique est successivement visible puis moins (ou plus du tout) par un sujet l'observant, selon des cycles successifs de longueur totale T opérés à la fréquence Fd prédéterminée, ce qui permet d'accroitre la lisibilité du contenu observé pour un sujet présentant des troubles dyslexiques. Selon l'invention, les périodes d'ouverture successives T1 de l'espace de vision ont chacune une durée comprise dans un intervalle de valeurs allant de 15 à 35% de la durée T des cycles.

La fréquence Fd des cycles (comprenant chacun une période d'ouverture de l'espace de vision et une période de fermeture de l'espace de vision) est comprise entre 60 et 90 Hz.

Selon un mode de réalisation, la fréquence Fd est fixe.

Selon un autre mode de réalisation, la fréquence Fd varie dans le temps.

Selon un mode de réalisation particulier, la fréquence Fd varie en croissant par pas successifs jusqu'à une valeur maximale selon une première vitesse, dite vitesse de croissance, puis varie en décroissant par pas successifs jusqu'à une valeur minimale selon une seconde vitesse, dite vitesse de décroissance, les variations croissantes et décroissantes se répétant itérativement dans le temps.

Selon un mode de réalisation particulier, la vitesse de décroissance et égale à la vitesse de croissance.

Selon un mode de réalisation particulier, les pas successifs sont de durées égales.

Selon un autre mode de réalisation particulier, les pas successifs varient en durée de sorte que la valeur de ladite fréquence Fd évolue selon une forme d'onde en triangle, en scie, ou en sinusoïde entre ladite valeur maximale et ladite valeur minimale.

Avantageusement la durée T1 des périodes d'activation varie dans le temps en évoluant de façon continue ou discontinue entre des valeurs bornes allant de 15 à 35% de la durée T des cycles. Il est entendu ici par de « façon continue » une évolution par incrément des pas successifs de durées égales.

Le phénomène de wobulation ainsi créé et appliqué à la fréquence Fd prédéterminée (variation de la fréquence Fd) permet de balayer un grand nombre de fréquences entre 60 Hz et 90 Hz, dont certaines seront plus efficaces pour l'aide à la lecture. Ces fréquences plus efficaces varient selon le sujet dyslexique. En balayant toutes les fréquences entre 60 et 90 Hz, le dispositif de l'invention ne nécessite aucun réglage préalable et devient efficace pour un grand nombre d'utilisateurs.

Un même avantage découle des variations de la durée T1 des périodes d'ouverture de l'espace de vision.

Ce phénomène de wobulation permet ainsi et dans certains cas, de réduire plus encore des ennuis liés à des troubles dyslexiques.

L'invention ne se limite pas aux seuls modes de réalisation décrits ci-avant, mais s'applique à tout dispositif optique de filtrage de la lumière reçue, adapté à l'observation d'un contenu graphique et/ou textuel sur un support quelconque (écran ou livre, par exemple), mettant en œuvre des opérations successives d'ouverture d'un espace de vision et de fermeture de ce même espace de vision, périodiquement, selon des cycles successifs opérés à une fréquence Fd prédéterminée entre 60 Hz et 90 Hz telle que les périodes d'ouverture T1 successives ont chacune une durée comprise dans un intervalle de valeurs allant de 15 à 35% de la durée T des cycles opérés. Par exemple et selon une variante, l'ouverture et la fermeture de l'espace de vision ou du plan de vision peut être réalisée par une mise en œuvre d'un ou plusieurs éléments mécaniques contrôlés selon la méthode décrite (fréquence Fd et rapport cyclique T1).

## Revendications

1. Dispositif optique (FILT) pour faciliter la lecture d'un contenu graphique et/ou textuel sur un support quelconque par des sujets dyslexiques, ledit dispositif comprenant une unité de contrôle (CTRLU) et un module de filtrage (LIMOD) adapté à l'ouverture et à la fermeture d'un espace de vision dans un spectre de lumière visible, ledit dispositif optique (FILT) étant configuré pour ouvrir et fermer périodiquement ledit espace de vision selon des cycles successifs opérés à une fréquence Fd, chaque cycle ayant une durée T et comprenant une période d'ouverture de l'espace de vision de durée T1 suivie ou précédée d'une période de fermeture de l'espace de vision de durée T2,
**caractérisé en ce que** ladite fréquence Fd est comprise dans un intervalle de valeurs allant de 60 à 90 Hz et la durée T1 des périodes d'ouverture de l'espace de vision est comprise dans un intervalle de valeurs allant de 15 à 35% de la durée T des cycles.

2. Dispositif optique selon la revendication 1, **caractérisé en ce que** ladite fréquence Fd est comprise dans un intervalle allant de 70 à 85 Hz et que la durée T1 des périodes d'ouverture de l'espace de vision est comprise dans un intervalle de valeurs allant de 22 à 32% de la durée T des cycles.

3. Dispositif optique selon la revendication 1 ou 2, **caractérisé en ce que** la fréquence Fd varie dans le temps.

4. Dispositif optique selon la revendication 3, **caractérisé en ce que** la fréquence Fd varie en croissant par pas successifs jusqu'à une valeur maximale selon une première vitesse, dite vitesse de croissance, puis varie en décroissant par pas successifs jusqu'à une valeur minimale selon une seconde vitesse, dite vitesse de décroissance, les variations croissantes et décroissantes se répétant itérativement dans le temps.

5. Dispositif optique selon la revendication 4 **caractérisé en ce que** ladite vitesse de décroissance et égale à ladite vitesse de croissance.

6. Dispositif optique selon la revendication 5, **caractérisé en ce que** lesdits pas successifs sont de durées égales.

7. Dispositif optique selon la revendication 5, **caractérisé en ce que** lesdits pas successifs varient en durée de sorte que la valeur de ladite fréquence Fd évolue selon une forme d'onde en triangle, en scie, ou en sinusoïde entre ladite valeur maximale et ladite valeur minimale.

8. Dispositif optique selon l'une quelconque des revendications précédentes caractérisé en que la durée T1 desdites des périodes d'ouverture de l'espace de vision varie dans le temps.

9. Dispositif optique (FILT) selon l'une quelconque des revendications 1 à 8, **caractérisé en ce que** ledit module de filtrage (LIMOD) comprend des éléments polarisants et des éléments de type cristaux liquides adaptés à une polarisation de la lumière reçue, transmise via ledit espace de vision.

10. Dispositif optique (FILT) selon la revendication 9, **caractérisé en ce que** ladite polarisation de la lumière reçue via ledit espace de vision est une polarisation verticale.

11. Dispositif selon l'une quelconque des revendications précédentes **caractérisé en ce qu'**il est d'un type compris dans la liste : une paire de lunettes, un casque, un masque, un écran intercalaire, un plan filtrant configuré pour être disposé devant ou contre un écran de restitution.

12. Dispositif selon l'une quelconque des revendications précédentes **caractérisé en ce que** ladite fréquence prédéterminée est définie par un utilisateur dudit dispositif.

13. Méthode de filtrage optique pour faciliter la lecture d'un contenu graphique et/ou textuel sur un support quelconque par des sujets dyslexiques, ladite méthode étant mis en œuvre dans un dispositif optique (FILT) comprenant une unité de contrôle (CTRLU) et un module de filtrage (LIMOD) adapté à l'ouverture et à la fermeture d'un espace de vision dans un spectre de lumière visible, ladite méthode comprenant des ouvertures et fermetures périodiques d'un espace de vision par cycles successifs opérés à une fréquence Fd prédéterminée, chaque cycle ayant une durée T et comprenant une période d'ouverture de l'espace de vision de durée T1 suivie ou précédée d'une période de fermeture de l'espace de vision de durée T2,
**caractérisée en ce que** ladite fréquence Fd est comprise dans un intervalle de valeurs allant de 60 à 90 Hz et **en ce que** la durée T1 des périodes d'ouverture de l'espace de vision est comprise dans un intervalle de valeurs allant de 15 à 35% de la durée T des cycles.

14. Dispositif optique selon la revendication 13, **caractérisé en ce que** ladite fréquence Fd est comprise dans un intervalle allant de 70 à 85 Hz et que la durée T1 des périodes d'ouverture de l'espace de vision est comprise dans un intervalle de valeurs allant de 22 à 32% de la durée T des cycles.

## Patentansprüche

1. Optische Vorrichtung (FILT) zum Erleichtern des Lesens eines grafischen und/oder textuellen Inhalts auf einem beliebigen Träger durch Legastheniker, die Vorrichtung umfassend eine Steuereinheit (CTRLU) und ein Filtermodul (LIMOD), das zum Öffnen und Schließen eines Sichtraums in einem Spektrum von sichtbarem Licht angepasst ist, wobei die optische Vorrichtung (FILT) zum periodischen Öffnen und Schließen des Sichtraums gemäß aufeinanderfolgenden Zyklen konfiguriert ist, die mit einer Frequenz Fd betrieben werden, wobei jeder Zyklus eine Dauer T aufweist und eine Öffnungsperiode des Sichtraums der Dauer T1 umfasst, gefolgt von oder vorangegangen durch eine Schließperiode des Sichtraums der Dauer T2,
**dadurch gekennzeichnet, dass** die Frequenz Fd in einem Wertebereich von 60 bis 90 Hz liegt und die Dauer T1 der Öffnungsperioden des Sichtbereichs in einem Wertebereich von 15 bis 35 % der Dauer T der Zyklen liegt.

2. Optische Vorrichtung nach Anspruch 1, **dadurch gekennzeichnet, dass** die Frequenz Fd in einem Bereich von 70 bis 85 Hz liegt und dass die Dauer T1 der Öffnungsperioden des Sichtbereichs in einem Wertebereich von 22 bis 32 % der Dauer T der Zyklen liegt.

3. Optische Vorrichtung nach Anspruch 1 oder 2,
**dadurch gekennzeichnet, dass** die Frequenz Fd zeitlich variiert.

4. Optische Vorrichtung nach Anspruch 3, **dadurch gekennzeichnet, dass** die Frequenz Fd in aufeinanderfolgenden Schritten bis zu einem Maximalwert gemäß einer ersten Geschwindigkeit, der Anstiegsgeschwindigkeit, ansteigend variiert, dann in aufeinanderfolgenden Schritten bis zu einem Minimalwert gemäß einer zweiten Geschwindigkeit, der Abfallgeschwindigkeit, abfallend variiert, wobei sich die ansteigenden und abfallenden Variationen iterativ mit der Zeit wiederholen.

5. Optische Vorrichtung nach Anspruch 4, **dadurch gekennzeichnet, dass** die Abfallgeschwindigkeit gleich der Anstiegsgeschwindigkeit ist.

6. Optische Vorrichtung nach Anspruch 5, **dadurch gekennzeichnet, dass** die aufeinanderfolgenden Schritte von gleicher Dauer sind.

7. Optische Vorrichtung nach Anspruch 5, **dadurch gekennzeichnet, dass** die aufeinanderfolgenden Schritte in ihrer Dauer variieren, sodass sich der Wert der Frequenz Fd in einer Dreiecks-, Sägezahn- oder Sinuswellenform zwischen dem Maximalwert und dem Minimalwert bewegt.

8. Optische Vorrichtung nach einem der vorstehenden Ansprüche, **dadurch gekennzeichnet, dass** die Dauer T1 der Öffnungsperioden des Sichtraums zeitlich variiert.

9. Optische Vorrichtung (FILT) nach einem der Ansprüche 1 bis 8, **dadurch gekennzeichnet, dass** das Filtermodul (LIMOD) polarisierende Elemente und Elemente von einem Flüssigkristalltyp umfasst, die für eine Polarisation des empfangenen Lichts angepasst sind, das über den Sichtraum übertragen wird.

10. Optische Vorrichtung (FILT) nach Anspruch 9,
**dadurch gekennzeichnet, dass** die Polarisation des Lichts, das über den Sichtraum empfangen wird, eine vertikale Polarisation ist.

11. Vorrichtung nach einem der vorstehenden Ansprüche,
**dadurch gekennzeichnet, dass** sie von einem Typ ist, der in der Liste enthalten ist: eine Brille, ein Helm, eine Maske, ein Zwischenschirm, eine Filterebene, die zum Angeordnetwerden vor oder an einem Wiedergabebildschirm konfiguriert ist.

12. Vorrichtung nach einem der vorstehenden Ansprüche,
**dadurch gekennzeichnet, dass** die vorbestimmte Frequenz durch einen Benutzer der Vorrichtung definiert wird.

13. Optisches Filterverfahren zum Erleichtern des Lesens eines grafischen und/oder textuellen Inhalts auf einem beliebigen Träger durch Legastheniker, wobei das Verfahren in einer optischen Vorrichtung (FILT) ausgeführt wird, umfassend eine Steuereinheit (CTRLU) und ein Filtermodul (LIMOD), das zum Öffnen und Schließen eines Sichtraums in einem Spektrum von sichtbarem Licht ausgebildet ist, das Verfahren umfassend periodische Öffnungen und Schließungen eines Sichtraums durch aufeinanderfolgende Zyklen, die mit einer vorbestimmten Frequenz Fd durchgeführt werden, wobei jeder Zyklus eine Dauer T aufweist und eine Öffnungsperiode des Sichtraums der Dauer T1 umfasst, auf die eine Schließperiode des Sichtraums der Dauer T2 folgt oder die dieser vorausgeht,
**dadurch gekennzeichnet, dass** die Frequenz Fd in einem Wertebereich von 60 bis 90 Hz liegt und **dass** die Dauer T1 der Öffnungsperioden des Sichtraums in einem Wertebereich von 15 bis 35 % der Dauer T der Zyklen liegt.

14. Optische Vorrichtung nach Anspruch 13, **dadurch gekennzeichnet, dass** die Frequenz Fd in einem Bereich von 70 bis 85 Hz liegt und dass die Dauer T1 der Öffnungsperioden des Sichtbereichs in einem Wertebereich von 22 bis 32 % der Dauer T der Zyklen liegt.

## Claims

1. An optical device (FILT) to facilitate the reading of graphic and/or textual content on any medium by individuals with dyslexia, said device comprising a control unit (CTRLU) and a filtering module (LIMOD) adapted to open and close a viewing space within a visible light spectrum, said optical device (FILT) being configured to periodically open and close said viewing space in successive cycles operated at a frequency Fd, each cycle having a duration T and comprising a period of duration T1 during which the viewing space is open, followed or preceded by a period of duration T2 during which the viewing space is closed,
**characterized in that** said frequency Fd falls within a range of values from 60 to 90 Hz, and the duration T1 of the periods during which the viewing space is open falls within a range of values from 15 to 35% of the cycle duration T.

2. The optical device according to claim 1, **characterized in that** said frequency Fd lies within a range from 70 to 85 Hz **and in that** the duration T1 of the periods during which the viewing space is open lies within a range of values from 22 to 32% of the cycle duration T.

3. The optical device according to claim 1 or 2, **characterized in that** the frequency Fd varies over time.

4. The optical device according to claim 3, **characterized in that** the frequency Fd varies by increasing in successive steps to a maximum value according to a first speed, referred to as increase speed, and then varies by decreasing in successive steps to a minimum value according to a second speed, referred to as decrease speed, the increasing and decreasing variations repeating iteratively over time.

5. The optical device according to claim 4, **characterized in that** said decrease speed is equal to said increase speed.

6. The optical device according to claim 5, **characterized in that** said successive steps are of equal durations.

7. The optical device according to claim 5, **characterized in that** said successive steps vary in duration such that the value of said frequency Fd changes according to a triangular, sawtooth or sinusoidal waveform between said maximum value and said minimum value.

8. The optical device according to any of the preceding claims, **characterized in that** the duration T1 of said periods during which the viewing space is open varies over time.

9. The optical device (FILT) according to any of claims 1 to 8, **characterized in that** said filtering module (LIMOD) comprises polarizing elements and liquid-crystal-type elements able to polarize the received light, transmitted through said viewing space.

10. The optical device (FILT) according to claim 9, **characterized in that** said polarization of the light received through said viewing space is vertical polarization.

11. The device according to any of the preceding claims,
**characterized in that** it is of a type included in the following list: a pair of glasses, a helmet, a mask, an interlayer screen, a filtering plane configured to be positioned in front of or against a display screen.

12. The device according to any of the preceding claims,
**characterized in that** said predetermined frequency is defined by a user of said device.

13. An optical filtering method to facilitate the reading of graphic and/or textual content on any medium by individuals with dyslexia, said method being implemented in an optical device (FILT) comprising a control unit (CTRLU) and a filtering module (LIMOD) adapted to open and close a viewing space within a visible light spectrum, said method comprising periodic opening and closing of a viewing space in successive cycles operated at a predetermined frequency Fd, each cycle having a duration T and comprising a period of duration T1during which the viewing space is open, followed or preceded by a period of duration T2 during which the viewing space is closed,
**characterized in that** said frequency Fd falls within a range of values from 60 to 90 Hz, **and in that** the duration T1 of the periods during which the viewing space is open falls within a range of values from 15 to 35% of the cycle duration T.

14. The optical device according to claim 13, **characterized in that** said frequency Fd falls within a range from 70 to 85 Hz **and in that** the duration T1 of the periods during which the viewing space is open falls within a range of values from 22 to 32% of the cycle duration T.
